# EUROPEAN PATENT APPLICATION

(11) **EP 4 119 163 A1**
(43) Date of publication of application: **18.01.2023**
(21) Application number: 21768657.5
(22) Date of filing: 12.03.2021
(51) Int. Cl.: A61K 45/00, A61K 31/235, A61P 31/12, A61P 43/00, C12Q 1/02, C12Q 1/70

(54) **VIRAL INFECTION THERAPEUTIC**

(30) Priority: 13.03.2020 US 202062988962 P; 01.06.2020 US 202063032758 P; 11.08.2020 JP 2020135519
(71) Applicant: The University of Tokyo, Bunkyo-ku, Tokyo 113-8654 (JP)
(72) Inventor: INOUE Jun-ichiro, Tokyo 113-8654 (JP); KAWAGUCHI Yasushi, Tokyo 113-8654 (JP); GOHDA Jin, Tokyo 113-8654 (JP); YAMAMOTO Mizuki, Tokyo 113-8654 (JP); SETO Yasuyuki, Tokyo 113-8654 (JP); DOI Kent, Tokyo 113-8654 (JP); MORIYA Kyoji, Tokyo 113-8654 (JP); MIYAZONO Kohei, Tokyo 113-8654 (JP); KAWAOKA Yoshihiro, Tokyo 113-8654 (JP); KISO Maki, Tokyo 113-8654 (JP)
(74) Representative: Isarpatent
(86) International application number: PCT/JP2021/009968
(87) International publication number: WO 2021/182597

(57) **Abstract**

The present invention relates to an inhibitory agent of membrane fusion between a virus envelope and a cell membrane of a host cell for the virus, wherein the inhibitory agent comprises a serine protease inhibitor, and a therapeutic agent or a therapeutic composition, comprising the inhibitory agent. More specifically, the present invention relates to the inhibitory agent, wherein the serine protease inhibitor is one or more of a compound represented by the following general formula (I) or a salt thereof, or a solvate or hydrate thereof, and a therapeutic agent or a therapeutic composition, comprising the inhibitory agent: 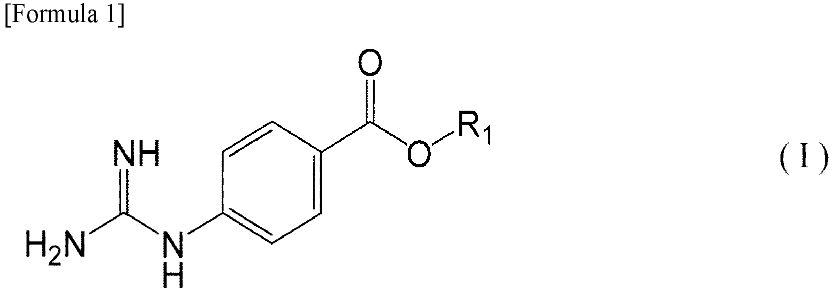 wherein R₁ represents a substituted or unsubstituted lower alkyl group, a substituted or unsubstituted lower alkenyl group, a substituted or unsubstituted aromatic hydrocarbon group, or a substituted or unsubstituted aromatic heterocyclic group, each of which may optionally comprise a heteroatom.

## Description

### Technical Field

The present invention relates to a therapeutic agent and a therapeutic composition for viral infection, such as, for example, COVID-19 (Coronavirus Disease 2019), and the like.

### Background Art

A patient with infection (COVID-19) caused by severe acute respiratory syndrome coronavirus 2 (SARS-CoV-2) was found in Wuhan (China) at the end of Year 2019, and then, this infection has spread around the world over two months. The cumulative number of infected people was 7,273,958, and the number of deaths was 413,372 (as of June 12, 2020). Even in Japan, the cumulative number of infected people was 17,332, and the number of deaths was 922 (as of June 12, 2020). It is said that a majority of infected people are asymptomatic carriers, and that 80% of people with symptoms are people with minor symptoms. However, when the symptoms become severe, or when the infected people are aged people or people with underlying disease, the infection would lead to death.

Clinical trials regarding the therapeutic effects of several existing pharmaceutical agents on COVID-19 are in progress. For example, it has been reported that SARS-CoV-2 is removed at high frequency from the nasopharynx by administration of Hydroxychloroquine that is an antimalarial drug (Non Patent Literature 1). On the other hand, with regard to anti-human immunodeficiency virus drugs (lopinavir and ritonavir) that had exhibited inhibitory activity against SARS-CoV *in vitro,* these drugs could not improve the symptoms of COVID-19 in randomized controlled trials (Non Patent Literature 2).

A pyrazinecarboxamide derivative, favipiravir, induces a lethal mutation to viral RNA-dependent RNA polymerase of Influenza A virus subtype H1N1, and thereby inhibits the activity thereof (Non Patent Literatures 3 and 4). It has been reported that favipiravir exhibits antiviral activity against RNA viruses such as norovirus, bunyavirus, arenavirus, flavivirus and filovirus (Non Patent Literatures 5 and 6), and thus, it is expected that favipiravir will exhibit antiviral activity against SARS-CoV-2.

Coronavirus is one type of envelope virus, the genomic RNA of which is surrounded with an envelope (outer membrane) (Non Patent Literature 7). Specific examples of the known coronavirus may include SARS-CoV, SARS-CoV-2, and MERS-CoV (Middle East respiratory syndrome coronavirus).

The invasion process of the coronavirus into host cells consists of two steps. In a first step, a spike protein (S protein) located in a virus envelope is divided into an S1 protein and an S2 protein by protease existing in host cells, and the S1 protein then binds to a receptor on a host cell membrane. SARS-CoV and SARS-CoV-2 use angiotensin converting enzyme 2 (ACE2) as a receptor, whereas MERS-CoV uses, as a receptor, CD26 that is different from ACE2. In a second step, the S2 protein is cleaved by transmembrane serine protease 2 (TMPRSS2) present on the cell surface (wherein this action is referred to as "priming"), and a fusion peptide existing in the S2 protein is thereby exposed and binds to the host cell membrane. As a result, membrane fusion between the virus envelope and the host cell membrane is induced (hereinafter, in the present description, this action is simply referred to as "membrane fusion" at times). By this membrane fusion, the viral RNA invades the cytoplasm, and replication of the viral genome occurs therein. In an experiment using mice, by knocking out TMPRSS2, the spread of infection of SARS-CoV and MERS-CoV in the respiratory tract was reduced, and the severity of the lung lesions was alleviated (Non Patent Literature 7).

Based on the aforementioned respects, it is considered that TMPRSS2 and ACE2 or CD26 play an extremely important role in coronaviral infection *in vivo* and the onset of the disease caused thereby.

To date, the present inventors have reported that a serine protease inhibitor, nafamostat mesylate (i.e. nafamostat mesilate), known as a therapeutic agent for acute pancreatitis and disseminated intravascular coagulation (DIC), effectively inhibits membrane fusion caused by the S protein of MERS-CoV (Non Patent Literature 8). In addition, it has been recently reported that ACE2 and TMPRSS2 are essential for infection with SARS-CoV-2 (Non Patent Literature 9), and thus, it has been expected that such membrane fusion caused by SARS-CoV-2 would be also inhibited by nafamostat mesylate. However, since receptor molecules used in the membrane fusion are totally different between MERS-CoV and SARS-CoV-2 (wherein ACE2 is used as a receptor in the case of SARS-CoV-2, whereas CD26 is used as a receptor in the case of MERS-CoV), whether or not nafamostat mesylate targets TMPRSS2 even in the case of SARS-CoV-2 is unknown.

Moreover, recently, Wang et al. have reported that nafamostat mesylate suppresses, to a certain extent, infection of Vero E6 cells with SARS-CoV-2, wherein, in the Vero E6 cells, TMPRSS2 is not expressed, but that the nafamostat mesylate exhibits its effects at a high concentration such as a 50% infection inhibitory concentration (IC₅₀) = 22.5 µM (which is referred to as " EC₅₀" in the publication) (Non Patent Literature 11). However, these results suggest that the effective concentration of nafamostat mesylate is precited to be a high concentration of several tens of µM or more, and thus that nafamostat mesylate is not likely to be suitable as a therapeutic agent for COVID-19.

### Citation List

### Non Patent Literature

Non Patent Literature 1: Gautret et al., Int J Antimicrob Agents 2020, 20, 105949, doi: 10.1016/j.ijantimicag.
Non Patent Literature 2: Cao et al., N Engl J Med 2020, 382, 1787-99, doi: 10.1056/NEJMoa2001282.
Non Patent Literature 3: Jin et al., PLoS One 2013, 8, e68347, doi: 10.1371/journal.pone.0068347
Non Patent Literature 4: Baranovich et al., J Virol 2013, 87, 3741-51, doi: 10.1128/JVI.02346-12
Non Patent Literature 5: Furuta et al., Antiviral Res 2013, 100, 446-54, doi: 10.1016/j.antiviral.2013.09.015.
Non Patent Literature 6: Jordan et al., Antivir Chem Chemother 2018, 26, 1-19, doi: 10.1177/2040206618764483.
Non Patent Literature 7: Iwata-Yoshikawa et al., J. Virol. 2019, 93, e01815-18, doi: 10.1128/JVI.01815-18.
Non Patent Literature 8: Yamamoto et al., Antimicrob Agents Chemother 2016, 60, 6532-6539, doi: 10.1128/AAC.01043-16.
Non Patent Literature 9: Hoffman et al., Cell 2020, 181, 271-280.e8, doi: 10.1016/j.cell.2020.02.052.
Non Patent Literature 10: Matsuyama et al., Proc Natl Acad Sci U S A 2020, 117, 7001-7003, doi: 10.1073/pnas.2002589117.
Non Patent Literature 11: Wang et al., Cell Res 2020, 30, 269-271, doi: 10.1038/s41422-020-0282-0.

### Summary of Invention

### Technical Problem

Considering the aforementioned circumstances, it is an object of the present invention to provide: an agent for inhibiting or suppressing viral infection, in particular, invasion of coronavirus (for example, SARS-CoV-2) or the like into cells that needs TMPRSS2 for its invasion into host cells; and a therapeutic agent and a therapeutic composition for viral infection, which comprise the aforementioned agent as an active ingredient.

In addition, it is another object of the present invention to provide a method for treating viral infection, using the above-described therapeutic agent or therapeutic composition.

### Solution to Problem

The present inventors have focused on the point that when the coronavirus invades host cells, it requires the activity of TMPRSS2 that is a serine protease, and have studied the effects of a TMPRSS2 inhibitory agent against the invasion of SARS-CoV-2 into host cells. The present inventors have utilized Dual Split Protein (DSP) reporter assay system, which is capable of quantitatively reproducing, in real time, membrane fusion characteristic of the invasion process of the coronavirus into host cells (Kondo et al., J Biol Chem 2010, 285, 14681-14688, doi: 10.1074/jbc.M109.067090: Ishikawa et al., Protein Eng Des Sel 2012, 25, 813-820, doi: 10.1093/protein/gzs051.), and have examined the effects of the above-described TMPRSS2 inhibitory agent. Not using a system of utilizing live virus, but using a membrane fusion system in which the DSP system is utilized, the influence of endocytosis (the pathway of influenza virus or the like to invade a host) can be eliminated. Therefore, a drug for inhibiting the cell invasion of coronavirus via TMPRSS2-dependent membrane fusion, wherein TMPRSS2 has been considered to play an extremely important role in coronaviral infection *in vivo* and the onset of the disease caused thereby according to Non Patent Literature 7, can be screened more precisely.

As such TMPRSS2 inhibitory agents, nafamostat mesylate (6-amidinonaphthalen-2-yl 4-guanidinobenzoate bis(methanesulfonate)) and camostat mesylate (i.e. camostat mesilate) (dimethylcarbamoylmethyl 4-(4-guanidinobenzoyloxy)phenylacetate monomethanesulfonate) were used. As target cells, human kidney-derived 293FT cells and human airway epithelium-derived Calu-3 cells and H3255 cells were used. As a result, it was revealed that membrane fusion is inhibited by all of these inhibitory agents. In particular, when the respiratory tract-derived Calu-3 cells and H3255 cells were used as target cells, nafamostat mesylate almost completely suppressed membrane fusion at a low concentration of 10 nM. These results show that the concentration of nafamostat mesylate used for the Calu-3 cells and the H3255 cells was about 10-fold lower than that used for the 293FT cells.

Furthermore, the present inventors have examined the influence of nafamostat mesylate on the *in vitro* infection of Calu-3 cells with SARS-CoV-2 (live virus), in addition to the DSP assay system. As a result, it was revealed that SARS-CoV-2 infection is suppressed by nafamostat mesylate, at an IC₅₀ value of about 10 nM according to the Tissue Culture Infectious Dose 50 (TCID50) method, and at low concentrations of IC₅₀ = 0.27 nM, IC₉₀ = 1.6 nM, IC₉₉ = 6.5 nM according to Quantitative-polymerase chain reaction (q-PCR) method.

The aforementioned results were surprising results that could not be predicted from the report of Wang et al. (Non Patent Literature 11). With regard to the pharmacokinetics of nafamostat mesylate, it has been reported that, when nafamostat mesylate was administered at a dose of 10 or 20 mg to healthy adult males via intravenous drip over 90 minutes, the blood concentration of unchanged agent became the maximum after 60 to 90 minutes after initiation of the drip, and the concentrations were 16.4 and 61.5ng/mL, respectively, and that when the present agent was administered to DIC patients via intravenous drip at a rate of 0.1mg/kg or 0.2mg/kg per hour for 13 to 23 days, a blood concentration of about 14 to 130 ng/mL was maintained (https://www.kegg.jp/medicus-bin/japic_med?japic_code = 00048710). Herein, 61.5 ng/mL corresponds to 114 nM, and 14 to 130 ng/mL correspond to 26 to 241 nM. Hence, the experimental results of the present inventors, namely, the results that nafamostat mesylate suppressed SARS-CoV-2 infection at low concentrations of IC₅₀ = 0.27 nM, IC₉₀ = 1.6 nM, and IC₉₉ = 6.5 nM, sufficiently suggest the possibility of the nafamostat mesylate as a therapeutic agent. In contrast, the value reported by Wang et al. that is IC₅₀ = 22.5 µM may deny the possibility of the nafamostat mesylate as a therapeutic agent for SARS-CoV-2 infection.

By the way, Wang et al. have used Vero E6 cells as cells infected with SARS-CoV-2. Vero E6 cells are a cell line established from the kidney of African green monkeys, which does not have tumorigenicity and deletes an interferon gene. Thus, an antiviral pathway cannot function in the cells, and the Vero E6 cells are suitable for virus experiments and vaccine manufacturing (Osada et al., DNA Res. 2014, 21, 673-683, doi: 10.1093/dnares/dsu029.: Govorkova et al., J. Virol. 1996, 70, 5519-5524), and the Vero E6 cells have been used over the world from a long time ago. However, the Vero E6 cells do not express TMPRSS2 and do not have a virus invasion pathway mediated by TMPRSS2-dependent membrane fusion, wherein TMPRSS2 is considered to play an extremely important role in coronaviral infection *in vivo* and the onset of the disease caused thereby. Taking into consideration the results of the DSP assay and also the fact that COVID-19 is a disease associated with respiratory organs, the present inventors have not used the Vero E6 cells, but have used a Calu-3 cell line that is derived from the airway epithelium and endogenously expresses TMPRSS2. As a result, the present inventors have succeeded in finding the excellent effects of nafamostat mesylate against COVID-19 for the first time.

Further, in addition to the aforementioned *in vitro* experiments, the present inventors have conducted a clinical trial regarding a combined administration of nafamostat mesylate and favipiravir to 11 patients suffering from severe COVID-19. Consequently, extremely favorable clinical trial results were obtained, such that 9 out of the 11 patients were discharged from ICU, and that 7 patients left the hospital.

The present invention has been completed based on the above-mentioned findings.

The outline of the present invention will be described below.

The present invention relates to an inhibitory agent of membrane fusion between a virus envelope and a cell membrane of a host cell for the virus, wherein the inhibitory agent comprises a serine protease inhibitor.

More specifically, the present invention relates to the inhibitory agent characterized in that the serine protease is one or more of a compound represented by the following general formula (I) or a salt thereof, or a solvate or hydrate thereof: 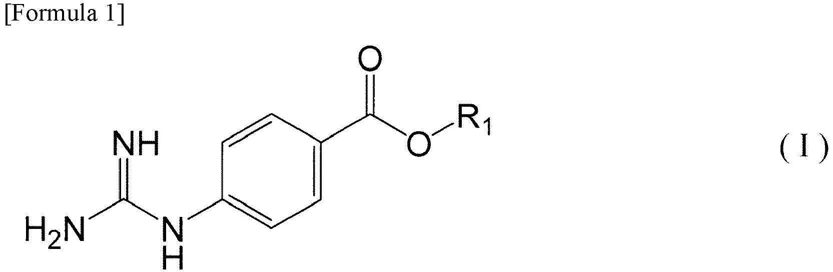 wherein R₁ represents a substituted or unsubstituted aromatic hydrocarbon group, a substituted or unsubstituted aromatic heterocyclic group, a substituted or unsubstituted lower alkyl group, or a substituted or unsubstituted lower alkenyl group, each of which may optionally comprise a heteroatom.

Further, the present invention relates to a therapeutic agent or a therapeutic composition for viral infection, wherein the therapeutic agent or the therapeutic composition comprises the above-described inhibitory agent. Furthermore, the present invention relates to a method for treating viral infection, comprising administering the therapeutic agent or the therapeutic composition to a patient.

Still further, the present invention relates to a method for evaluating the efficacy of an inhibitory agent of coronaviral infection, wherein the method is characterized in that a cell that is mainly TMPRSS2-dependently infected with the virus via a membrane fusion pathway is used as a host cell.

### Advantageous Effects of Invention

The therapeutic agent or therapeutic composition of the present invention exhibits excellent effects on the treatment of coronaviral infection, in particular, the treatment of infection with SARS-CoV-2.

The therapeutic agent or therapeutic composition of the present invention does not only suppress infection with viruses, in particular, infection with SARS-CoV-2, but it also exhibits effects to alleviate physical symptoms provoked after the infection, for example, various symptoms associated with pulmonary intravascular coagulopathy (PIC).

### Brief Description of Drawings

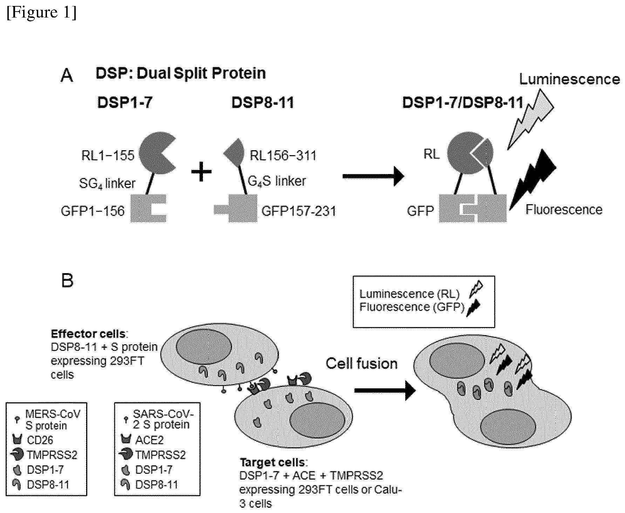

[Figure 1] Figure 1 shows an outline of a DSP assay. DSP1-7 has the structure of RL₁₋₁₅₅-Ser-Gly-Gly-Gly-Gly-GFP₁₋₁₅₆, and DSP8-11 has the structure of Met-GFP₁₅₇- ₂₃₁- Ser -Gly-Gly-Gly-Gly-RL₁₅₆₋₃₁₁. DSP1-7 and DSP8-11 efficiently form complexes, and after formation of the complexes, they emit luminescence and fluorescence signals, respectively (A). Effector cells (293FT cells expressing DSP8-11 and S protein) and target cells (293FT cells or Calu-3 cells expressing DSP1-7, ACE2 and TMPRSS2) are co-cultured. After completion of the cell fusion, DSP1-7 and DSP8-11 are re-associated with each other in the fused cells, so that GFP (fluorescence) and RL (luminescence) signals are generated (B).

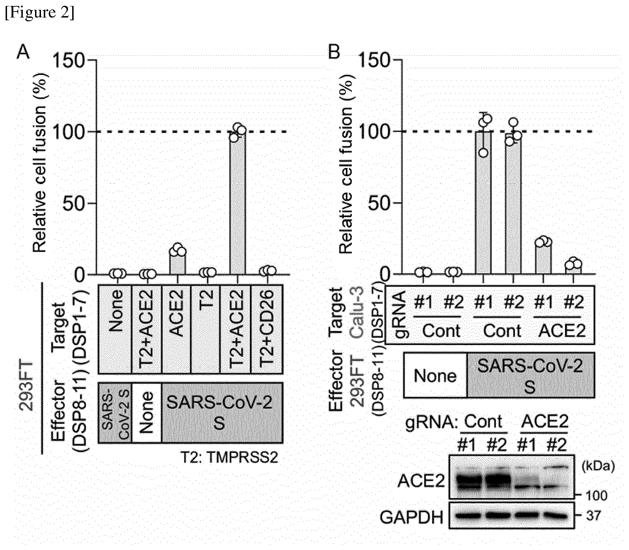

[Figure 2] Figure 2 shows the results obtained by studying the ACE2/TMPRSS2 dependence in membrane fusion between of SARS-CoV-2 S protein-expressing cells and ACE2- and TMPRSS2-expressing cells. 293FT cells were used as effector cells and target cells, and RL (renilla luciferase) activity generated from the co-culture was then measured. The relative cell fusion was calculated by setting the RL activity generated from the co-culture of the S protein-expressing cells and the cells expressing both the receptors ACE2 and TMPRSS2 to be 100%, and then standardizing RL activity generated from each co-culture with respect to the aforementioned RL activity (A). Figure 2B shows the results of a fusion assay, which was performed using 293FT cells as effector cells, and using wild-type Calu-3 cells or ACE2 knock out Calu-3 cells as target cells. ACE2 was knocked out by a CRISPR-Cas9 system using gRNA. The lower view shows the results of Western blotting that was performed in order to confirm the knocking out of ACE2 (B).

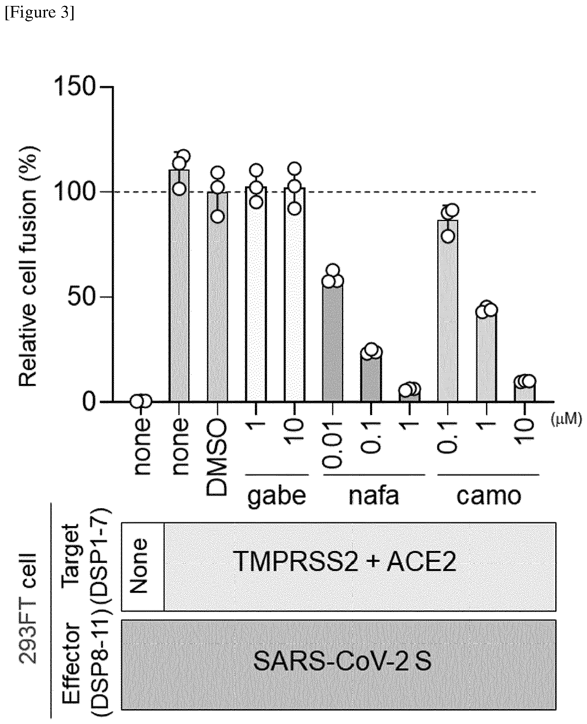

[Figure 3] Figure 3 shows the results obtained by studying the influence of 3 types of therapeutic agents for pancreatitis on the membrane fusion induced by a SARS-CoV-2 S protein. The influence of clinically used 3 types of therapeutic agents for pancreatitis on the membrane fusion was evaluated by a DSP assay. The relative cell fusion was calculated by setting the RL activity generated from the co-culture to which only DMSO was added to be 100%, and then standardizing RL activity generated from each co-culture with respect to the aforementioned RL activity. "gabe": gabexsate mesylate, "nafa": nafamostat mesylate, and "camo": camostat mesylate. The "gabe" and "nafa" are therapeutic agents for acute pancreatitis and disseminated intravascular coagulation (DIC), and the "camo" is a therapeutic agent for acute pancreatitis and reflux esophagitis.

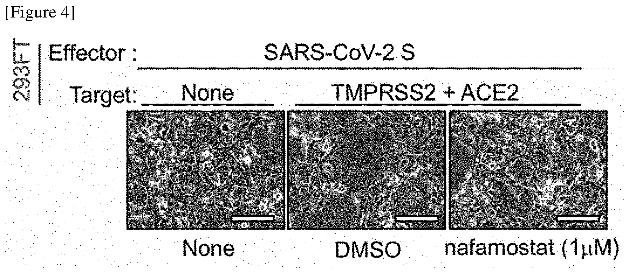

[Figure 4] Figure 4 is an image obtained by observing the cell fusion induced by the S protein of SARS-CoV-2 under a phase-contrast microscope. The scale bar indicates 100 µm.

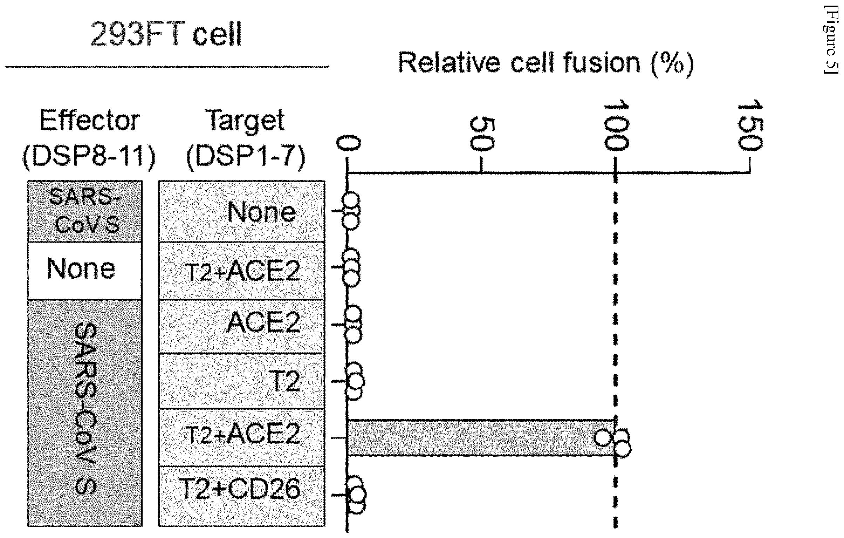

[Figure 5] Figure 5 shows the results obtained by studying the ACE2/TMPRSS2 dependence in membrane fusion between SARS-CoV S protein-expressing cells and ACE2- and TMPRSS2-expressing cells. 293FT cells were used as effector cells and target cells, and RL (renilla luciferase) activity generated from the co-culture was then measured. The relative cell fusion was calculated by setting the RL activity generated from the co-culture of the S protein-expressing cells and the cells expressing both the receptors ACE2 and TMPRSS2 to be 100%, and then standardizing RL activity generated from each co-culture with respect to the aforementioned RL activity.

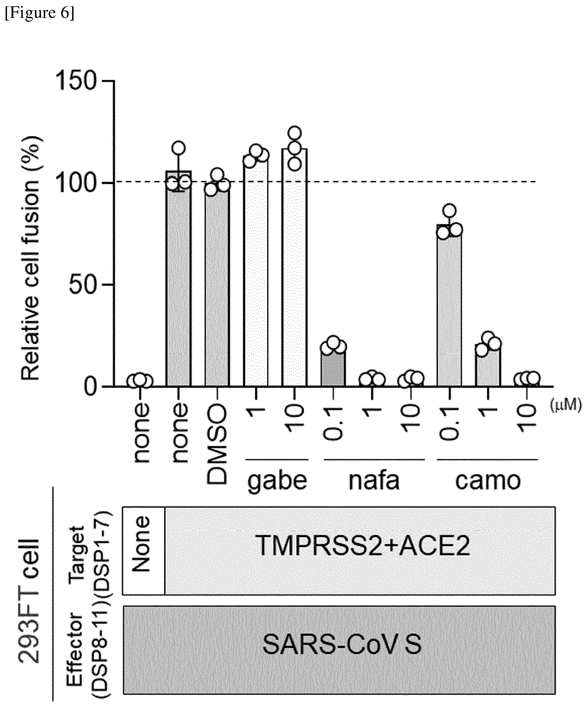

[Figure 6] Figure 6 shows the results obtained by studying the influence of 3 types of therapeutic agents for pancreatitis on the membrane fusion induced by a SARS-CoV S protein. The influence of clinically used 3 types of therapeutic agents for pancreatitis on the membrane fusion was evaluated by a DSP assay. The relative cell fusion was calculated by setting the RL activity generated from the co-culture to which only DMSO was added to be 100%, and then standardizing RL activity generated from each co-culture with respect to the aforementioned RL activity. "gabe": gabexsate mesylate, "nafa": nafamostat mesylate, and "camo": camostat mesylate. The "gabe" and "nafa" are therapeutic agents for acute pancreatitis and disseminated intravascular coagulation (DIC), and the "camo" is a therapeutic agent for acute pancreatitis and reflux esophagitis.

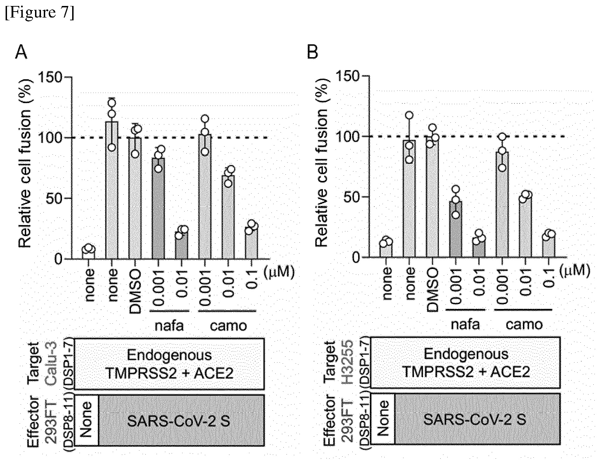

[Figure 7] Figure 7 shows the results obtained by studying the inhibitory effects of nafamostat mesylate and camostat mesylate against the membrane fusion induced by the S protein of SARS-CoV-2. These are the results of a DSP assay using Calu-3 cells (A) and H3255 cells (B) as target cells. "nafa": nafamostat mesylate, and "camo": camostat mesylate.

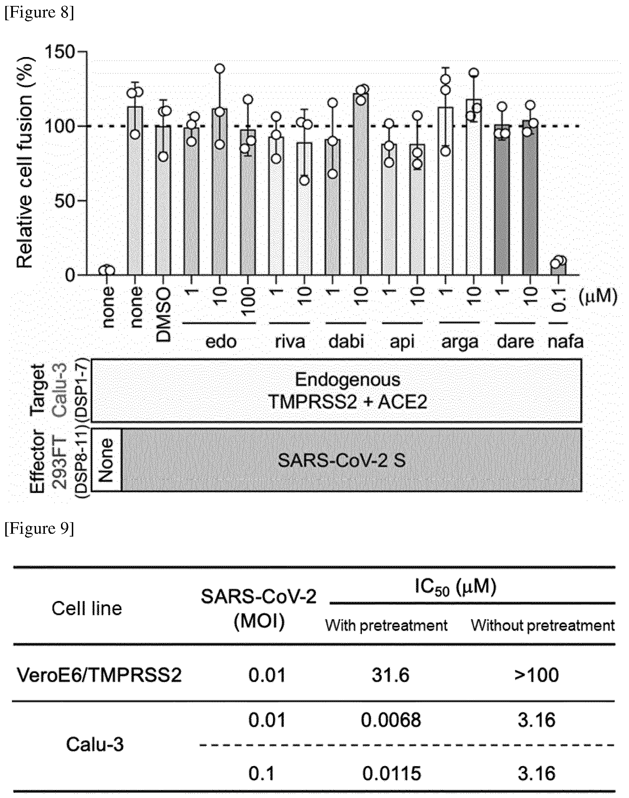

[Figure 8] Figure 8 shows the results obtained by studying the influence of various anticoagulants on the membrane fusion induced by the S protein of SARS-CoV-2. Using Calu-3 as target cells, a DSP assay was carried out. "edo": edoxaban, "riva": rivaroxaban, "dabi": dabigatran, "api": apixaban, "arga": argatroban, and "dare": darexaban.
[Figure 9] Figure 9 shows the results obtained by studying the inhibitory effects of nafamostat mesylate against infection of Calu-3 cells and VeroE6/TMPRSS2 cells with SARS-CoV-2 according to a TCID50 method. VeroE6/TMPRSS2 cells or Calu-3 cells were treated in the presence (with pretreatment) or absence (without pretreatment) of nafamostat mesylate (10-fold dilution series from 100 µM to 10 nM, 4 wells for each concentration) for 1 hour. After completion of the treatment, SARS-CoV-2 was added to the cells, and the obtained mixture was then incubated for 30 minutes. Thereafter, in the experiment with pretreatment, the medium was exchanged with a fresh medium containing nafamostat mesylate having the same concentration as that before viral infection. In the experiment without pretreatment, the medium was exchanged with a fresh medium containing nafamostat mesylate having 10-fold dilution series from 100 µM to 10 nM (4 wells for each concentration). In the case of the VeroE6/TMPRSS2 cells, IC₅₀ was calculated 3 days after the infection, and in the case of the Calu-3 cells, IC₅₀ was calculated 5 days after the infection. The experiment was carried out 4 times.

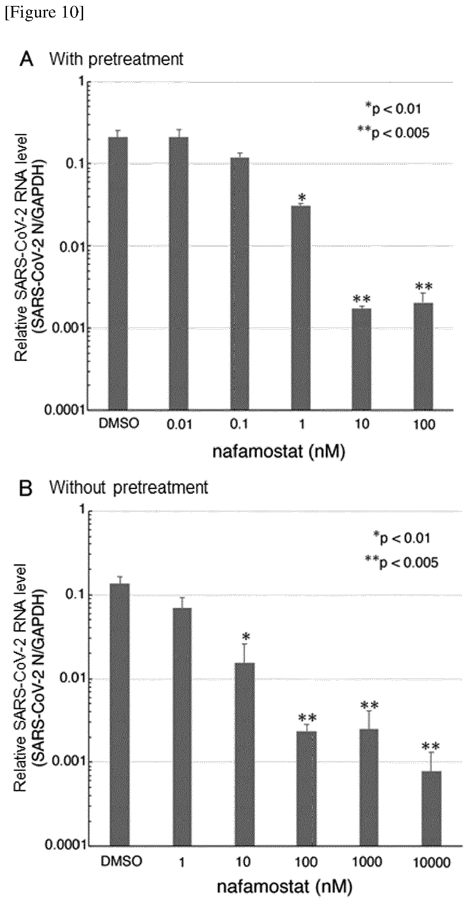

[Figure 10] Figure 10 shows the results obtained by studying the inhibitory effects of nafamostat mesylate against infection of Calu-3 cells with SARS-CoV-2 according to a qPCR method. Calu-3 cells were treated in the presence (with pretreatment) or absence (without pretreatment) of nafamostat mesylate (10-fold dilution series from 100 nM to 0.01 nM, 3 wells for each concentration) for 1 hour. After completion of the treatment, SARS-CoV-2 was added to the cells, and the obtained mixture was then incubated for 30 minutes. Thereafter, in the experiment with pretreatment, the medium was exchanged with a fresh medium containing nafamostat mesylate having the same concentration as that before viral infection. In the experiment without pretreatment, the medium was exchanged with a fresh medium containing nafamostat mesylate having 10-fold dilution series from 100 µM to 10 nM (3 wells for each concentration). The cells were recovered 24 hours after infection with the virus, and viral RNA was then quantified by a q-PCR method, and IC₅₀, IC₉₀ and IC₉₉ were then calculated.

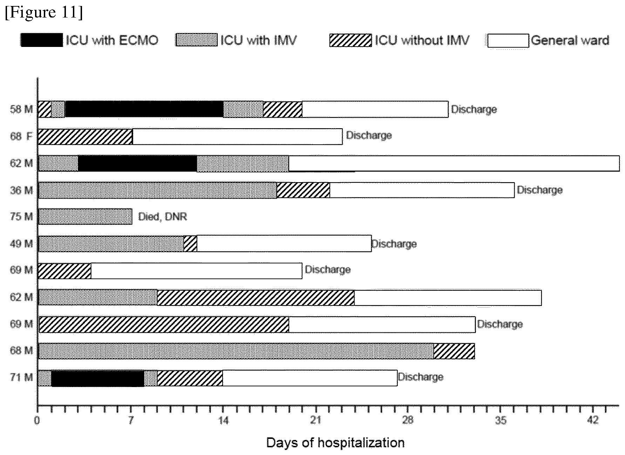

[Figure 11] Figure 11 shows the results of a combination therapy of nafamostat mesylate and favipiravir. In May 7, 2020, one patient died, but 7 patients were extubated. Observation of the progress was carried out on all of the patients for at least 33 days. The longitudinal axis indicates sex (F: female, M: male) and age. "ICU": intensive care unit, "ECMO": extracorporeal membrane oxygenation, "IMV": intermittent mandatory ventilation, and "DNR": do-not-resuscitate.

### Description of Embodiments

Hereinafter, the embodiments of the present invention will be described.

A first embodiment of the present invention relates to an inhibitory agent of membrane fusion between a virus envelope and a cell membrane of a host cell for the virus, wherein the inhibitory agent comprises a serine protease inhibitor (i.e. a proteolytic enzyme inhibitor) (hereinafter also referred to as "the inhibitory agent of the present invention").

Herein, the virus envelope means a coating consisting of a lipid and a glycoprotein, which is possessed by certain types of viruses, and the coating encloses a protein shell containing a gene therein, which is called "capsid." Examples of the known virus having such an envelope may include HIV-1 virus, influenza virus, and coronavirus. A typical virus in the embodiment of the present invention may be coronavirus.

The "virus" of the embodiment of the present invention is desirably a virus that needs the activity of TMPRSS2 (Transmembrane serine protease 2) that is a serine protease, when the virus invades a host cell as an infection target. Examples of such a virus may include SARS-CoV and SARS-CoV-2. In addition, in the embodiment of the present invention, the host cell means a cell that is infected with the virus, and it is, for example, a cell that is infected with the virus, mainly in a TMPRSS2-dependent manner, via a membrane fusion pathway. Examples of such a cell *in vivo* may include airway epithelial cells, and cells existing in various organs such as nasal cavity, intestine, and blood.

Examples of the serine protease inhibitor of the embodiment of the present invention may include one or more (a mixture of the compounds represented by the above general formula (I)) selected from the compound represented by the above general formula (I) or a salt thereof, or a solvate or hydrate thereof.

In the formula (I), R₁ is a substituted or unsubstituted lower alkyl group, a substituted or unsubstituted lower alkenyl group, a substituted or unsubstituted aromatic hydrocarbon group, or a substituted or unsubstituted aromatic heterocyclic group, each of which may optionally comprise a heteroatom. Herein, the "heteroatom" means an atom other than carbon and hydrogen, and examples of the heteroatom may include oxygen and nitrogen. The term "substituted or unsubstituted" means that the aforementioned group may have a substituent or may not be substituted. In particular, when the aromatic hydrocarbon group and the aromatic heterocyclic group have a substituent, the number of such substituents or the substituted position is not particularly limited.

Moreover, the "lower alkyl group" means a linear, branched or cyclic hydrocarbon containing 1 to 20, and preferably 1 to 10 carbon atoms, or a combination thereof.

The preferred R₁ may be an aromatic hydrocarbon group having a substituent, which is represented by the following formula (1) or (2):

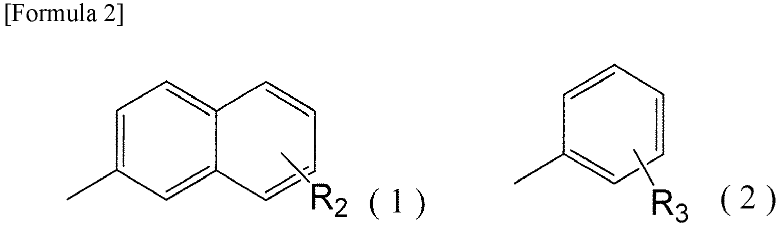

Herein, in the above formulae (1) and (2), R₂ and R₃ may each independently represent a lower alkyl group optionally having a heteroatom and/or a substituent. The preferred R₂ may be a substituent represented by the following formula (3), and the substituted position is preferably position 2 or position 6. The preferred R₃ may be a substituent represented by the following formula (4), and the substituted position is preferably position 4.

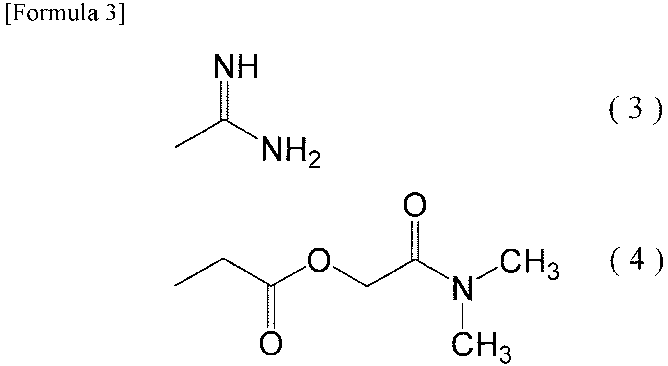

The salt of the compound represented by the general formula (I) may be a pharmaceutically acceptable salt. When an acidic group exists, examples of the salt may include: salts with alkali metals and alkaline earth metals such as lithium, sodium, potassium, magnesium, and calcium; salts with amines such as ammonia, methylamine, dimethylamine, trimethylamine, dicyclohexylamine, tris(hydroxymethyl)aminomethane, N,N-bis(hydroxyethyl)piperazine, 2-amino-2-methyl-1-propanol, ethanolamine, N-methylglucamine, and L-glucamine; and salts with basic amino acids such as lysine, δ-hydroxylysine, and arginine. When a basic group exists, examples of the salt may include: salts with mineral acids such as hydrochloric acid, hydrobromic acid, sulfuric acid, nitric acid, and phosphoric acid; salts with organic acids such as methanesulfonic acid (mesylic acid), benzenesulfonic acid, p-toluenesulfonic acid, acetic acid, propionic acid, tartaric acid, fumaric acid, maleic acid, malic acid, oxalic acid, succinic acid, citric acid, benzoic acid, mandelic acid, cinnamic acid, lactic acid, glycolic acid, glucuronic acid, ascorbic acid, nicotinic acid, and salicylic acid; and salts with acidic amino acids such as aspartic acid and glutamic acid.

Moreover, the compound represented by the general formula (I) includes stereoisomers thereof such as tautomers and enantiomers, unless otherwise stated. That is to say, when the compound represented by the general formula (I) comprises one or two or more asymmetric carbons, with regard to the stereochemistry of the asymmetric carbon(s), they each independently may adopt either an (R) form or an (S) form, and may be present as stereoisomers of the derivative, such as enantiomers or diastereomers.

Examples of the protease inhibitor comprised in the inhibitory agent of the present invention may include: nafamostat (6-amidinonaphthalen-2-yl 4-guanidinobenzoate) represented by the following formula (5), more specifically, nafamostat mesylate; and camostat (6-amidinonaphthalen-2-yl 4-guanidinobenzoate) represented by the following formula (6), more specifically camostat mesylate.

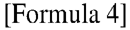

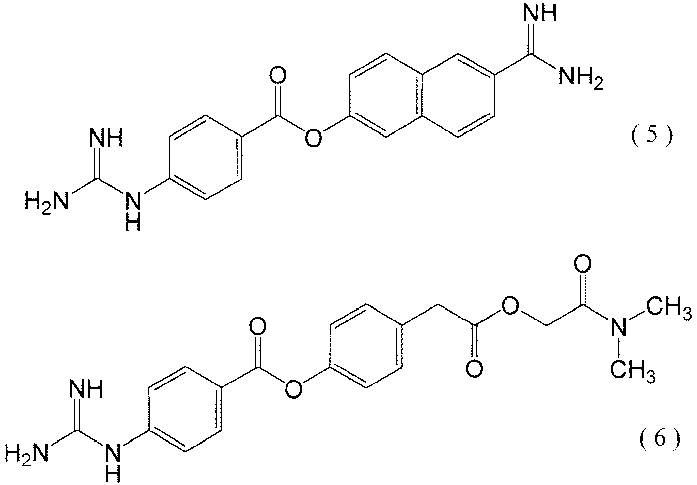

A second embodiment of the present invention relates to a therapeutic agent or a therapeutic composition for viral infection, wherein the therapeutic agent or the therapeutic composition comprises, as an active ingredient, the inhibitory agent according to the first embodiment of the present invention (hereinafter also referred to as "the therapeutic agent, etc. of the present invention"). Specifically, the second embodiment relates to a therapeutic agent or a therapeutic composition for viral infection, comprising, as an active ingredient, a serine protease inhibitor, in particular, a TMPRSS2 inhibitor. More specifically, the TMPRSS2 inhibitor may be one or more (a mixture of the compounds represented by the above general formula (I)) selected from the compound represented by the general formula (I) or a salt thereof, or a solvate or hydrate thereof. Examples of the preferred TMPRSS2 inhibitor may include, but are not particularly limited to, nafamostat (6-amidinonaphthalen-2-yl 4-guanidinobenzoate) represented by the above formula (5), more specifically, nafamostat mesylate, and camostat (6-amidinonaphthalen-2-yl 4-guanidinobenzoate) represented by the following formula (6), more specifically camostat mesylate.

The therapeutic agent for viruses, etc. according to the embodiment of the present invention is suitable for the treatment of infection with viruses that need the activity of TMPRSS2 as a serine protease, when the viruses invade the host cells as infection targets. Examples of such viruses may include, but are not particularly limited to, SARS-CoV and SAES-CoV-2. More typically, the virus may be SARS-CoV-2.

With regard to the therapeutic agent for viruses according to the embodiment of the present invention, the active ingredient (for example, the serine protease inhibitor, etc.) itself may be administered, but in general, the present therapeutic agent is desirably administered in the form of a therapeutic composition comprising one or two or more pharmaceutical additives, as well as one or more substances serving as an active ingredient(s). The therapeutic agent, etc. of the present invention may comprise a plurality of the inhibitory agents of the present invention as active ingredients. Furthermore, existing components that are recognized to have therapeutic effects on viruses may be mixed into the therapeutic agent, etc. of the present invention.

The dosage forms of the therapeutic agent, etc. of the present invention may include tablets, capsules, granules, powder agents, syrups, suspensions, suppositories, ointments, creams, gelling agents, patches, inhalants, and injections. These preparations are produced according to ordinary methods. Liquid preparation may be dissolved or suspended in water or another suitable solvent at the time of use. In addition, tablets and granules may be coated by a publicly known method. Injections are prepared by dissolving the active ingredient in water. As necessary, the active ingredient of the injection may be dissolved in a normal saline or a glucose solution, and further, a buffer agent or a preservative may be added to such a solution.

A preparation for use in oral administration or parenteral administration is provided in any given preparation form. Examples of the preparation form that can be prepared herein may include: therapeutic agents or therapeutic compositions for use in oral administrations, having forms such as granules, fine granules, powder agents, hard capsules, soft capsules, syrups, emulsions, suspensions, or liquid agents; and therapeutic agents or therapeutic compositions for use in parenteral administrations such as intravenous administration, intermuscular administration, or subcutaneous administration, having forms such as injections, drops, transdermal absorbents, transmucosal absorbents, nasal drops, inhalants, or suppositories. Such injections or drops can be prepared as powdery dosage forms such as freeze-dried forms, and can be then used by being dissolved in an appropriate aqueous medium such as a normal saline at the time of use.

The types of pharmaceutical additives used in production of the therapeutic agent, etc. of the present invention, the ratio of the pharmaceutical additives to the active ingredient, or a method for producing a pharmaceutical agent or a pharmaceutical composition can be appropriately selected by a person skilled in the art, depending on the forms thereof. As such pharmaceutical additives, inorganic or organic substances, or solid or liquid substances can be used. In general, such pharmaceutical additives can be mixed in an amount from 1% by weight to 90% by weight, based on the weight of the active ingredient. Specific examples of the pharmaceutical additives may include lactose, glucose, mannit, dextrin, cyclodextrin, starch, sucrose, magnesium aluminometasilicate, synthetic aluminum silicate, sodium carboxymethyl cellulose, hydroxypropyl starch, calcium carboxymethyl cellulose, ion exchange resin, methyl cellulose, gelatin, gum Arabic, hydroxypropyl cellulose, hydroxypropyl methyl cellulose, polyvinyl pyrrolidone, polyvinyl alcohol, light anhydrous silicic acid, magnesium stearate, talc, tragacanth, bentonite, veegum, titanium oxide, sorbitan fatty acid ester, sodium lauryl sulfate, glycerin, fatty acid glycerin ester, purified lanolin, glycerogelatin, polysorbate, macrogol, vegetable oil, wax, liquid paraffin, white petrolatum, fluorocarbon, nonionic surfactant, propylene glycol, and water.

In order to produce a solid preparation for use in oral administration, an active ingredient is mixed with excipient components, such as, for example, lactose, starch, crystalline cellulose, calcium lactate, or anhydrous silicic acid, to form a powder agent. Otherwise, as necessary, a binder such as white sugar, hydroxypropyl cellulose or polyvinyl pyrrolidone, a disintegrator such as carboxymethyl cellulose or calcium carboxymethyl cellulose, and the like are further added thereto, and the obtained mixture is then subjected to wet or dry granulation to form a granule. In order to produce a tablet, such a powder agent or a granule is directly used, or a lubricant such as magnesium stearate or talc is added thereto, and they are then subjected to tableting. Such a granules or a tablet can be coated with an enteric coating base material such as hydroxypropylmethyl cellulose phthalate or a methacrylic acid-methyl methacrylate polymer to form an enteric coated preparation. Otherwise, such a granule or tablet can be coated with ethyl cellulose, carnauba wax, or hydrogenated oil to form a prolonged action preparation. Moreover, in order to produce a capsule, a powder agent or a granule is filled into a hard capsule. Otherwise, an active ingredient is directly used, or is dissolved in glycerin, polyethylene glycol, sesame oil, olive oil, etc., and the obtained mixture is then coated with a gelatin film, so that a soft capsule can be prepared.

In order to produce an injection, an active ingredient is dissolved in distilled water for injection, together with, as necessary, a pH adjuster such as hydrochloric acid, sodium hydroxide, lactose, lactic acid, sodium, sodium monohydrogen phosphate or sodium dihydrogen phosphate, and a tonicity agent such as sodium chloride or glucose, and thereafter, the obtained solution is subjected to aseptic filtration, and is then filled into an ampoule. Otherwise, mannitol, dextrin, cyclodextrin, gelatin, etc. are further added to the obtained solution, and the thus mixed solution is then subjected to vacuum - freeze drying, so that the injection may be prepared as an injection that is dissolved at the time of use. Alternatively, lecithin, polysorbate 80, polyoxyethylene hydrogenated castor oil, etc. can be added to the active ingredient, and they can be then emulsified in water to prepare an emulsion for injection.

In order to produce an agent for rectal administration, an active ingredient may be humidified and dissolved, together with a suppository base material such as cacao butter, fatty acid tri-, di- and mono-glyceride, or polyethylene glycol, and thereafter, the obtained mixture may be poured into a mold and may be then cooled. Otherwise, an active ingredient may be dissolved in polyethylene glycol, soybean oil or the like, and the obtained mixture may be then coated with a gelatin film.

The applied dose and the number of doses of the therapeutic agent, etc. of the present invention are not particularly limited, and the applied dose and the number of doses can be selected, as appropriate, by a doctor's judgement, depending on conditions such as the purpose of prevention and/or treatment of deterioration/progression of a therapeutic target disease, the type of the disease, and the body weight, age, etc. of a patient.

In general, the daily dose of the present agent, etc. for an adult by oral administration is approximately 0.01 to 1000 mg (the weight of the active ingredient), and the present therapeutic agent, etc. can be administered once per day, or divided over several administrations per day. When the present therapeutic agent, etc. is used in the form of an injection, it is desired to continuously or intermittently administer the therapeutic agent or the like to an adult at a daily dose of 0.001 to 1000 mg (the weight of the active ingredient).

The therapeutic agent, etc. of the present invention may also be prepared in the form of a sustained release preparation, such as an implant and a delivery system encapsulated into a microcapsule, by using a carrier capable of preventing the immediate removal of the therapeutic agent from the body. Examples of such a carrier that can be used herein may include biodegradable and biocompatible polymers such as ethylene vinyl acetate, polyacid anhydride, polyglycolic acid, collagen, polyorthoester, and polylactic acid. Such materials can be easily prepared by a person skilled in the art. In addition, a liposome suspension can also be used as a pharmaceutically acceptable carrier. Such a liposome is not limited, but can be prepared as a lipid composition comprising phosphatidylcholine, cholesterol and PEG-induced phosphatidyl ethanol (PEG-PE), by being passed through a filter having a suitable pore size, so that it can have a size suitable for the use thereof, and thereafter, the lipid composition can be purified by a reversed phase evaporation method.

The therapeutic agent, etc. of the present invention may be provided in the form of a kit together with an instruction manual regarding an administration method and the like. The therapeutic agent included in the kit is supplied with a container produced with a material that effectively sustains the activity of structural components of the therapeutic agent, etc. for a long period of time, is not adsorbed on the inner side of the container, and does not degenerate the structural components. For instance, a sealed glass ampoule may include a buffer or the like, which is enclosed in the presence of neutral and non-reactive gas such as nitrogen gas.

Moreover, the kit may be included with an instruction manual. The instruction manual of the kit may be printed out on a paper or the like, or may be stored in an electromagnetically readable medium such as CD-ROM or DVD-ROM and may be then supplied to users.

A third embodiment of the present invention relates to a method for treating viral infection (for example, COVID-19, etc.), comprising administering the therapeutic agent, etc. of the present invention to a patient.

The term "to treat" means herein to stop or alleviate progression and deterioration of the pathological conditions induced in a mammal infected with the virus. The "mammal" as a therapeutic target means any given animal classified into Mammals. Examples of the mammal may include, but are not particularly limited to, humans, companion animals such as dogs, cats, rabbits, ferrets, mice and hamsters, and living stock animals such as bovines, pigs, sheep and horses. A particularly preferred "mammal" is a human.

When the viral infection is COVID-19, significant microvascular thrombosis and bleeding, which are associated with inflammation of the alveoli and the interstitium, are observed in the pathological condition image of a patient's lung. Such symptoms are referred to as "pulmonary intravascular coagulopathy (PIC)" (McGonagle et al., Lancet Rheumatology May 7, 2020 https://doi.org/10.1016/s2665-9913(20)30121-1.). In the early stage of PIC, differing from disseminated intravascular coagulation (DIC), a significant increase in the D-dimer value (which reflects pulmonary vascular thrombosis attended with abnormal fibrinolysis) and elevated cardiac enzyme levels (which reflect urgent vascular stress occurring due to lung hypertension) are observed. In particular, it has been reported that an increase in the D-dimer value may become an indicator to state that the disease becomes more severe (Zhang et al., J Thromb Haemost 19 April 2020 https://doi.org/10.1111/jth.14859: Petrilli et al., Brit Med J 22 May 2020 https://www.bmj.com/content/369/bmj.m1966), and also that an increase in the D-dimer value that is more than 1 µg/mL increases 18-fold the odds ratio for death (McGonagle et al., as mentioned above).

The therapeutic agent, etc. of the present invention comprising, as an active ingredient, nafamostat having anticoagulant action or a salt thereof, is considered to be extremely effective for alleviation of various symptoms of PIC. When the D-dimer value serving as an indicator of the severity of a COVID-19 patient is, for example, higher than 1 µg/mL, initiation of administration of the therapeutic agent, etc. of the present invention comprising the nafamostat or a salt thereof as an active ingredient to the patient in an early stage is considered to be effective even for the improvement of prognosis.

In addition, the therapeutic agent, etc. of the present invention comprising, as an active ingredient, a TMPRSS2 inhibitor (for example, nafamostat or a salt thereof, camostat or a salt thereof, etc.) has, as a point of action, the cell invasion of SARS-CoV and SARS-CoV-2 mediated by membrane fusion. Thus, when the therapeutic agent of the present invention is used, for example, in combination with other therapeutic agents, a combination of the therapeutic agent of the present invention with a therapeutic agent having a different point of action, for example, with a therapeutic agent having, as a point of action, replication of SARS-CoV, SARS-CoV-2 and the like in host cells, is considered to be effective. Examples of such other therapeutic agents may include favipiravir, Remdesivir (Grein et al., N Engl J Med, doi: 10.1056/NEJMoa2007016), and Nelfinavir (Ohashi et al., BioRxiv DOI 10.1101/2020.04.14.039925).

A fourth embodiment of the present invention relates to: use of a serine protease inhibitor for production of an inhibitory agent of membrane fusion between a virus envelope and a cell membrane of a host cell for the virus; or use of a serine protease inhibitor for production of a therapeutic agent or a therapeutic composition for viral infection. For details of the present embodiment, please refer to explanation regarding the first and second embodiments.

A fifth embodiment of the present invention relates to a method for evaluating the efficacy of an inhibitory agent of coronaviral infection, wherein the method is characterized in that a cell that is mainly TMPRSS2-dependently infected with the virus via a membrane fusion pathway is used as a host cell.

Taking into consideration the fact that the coronaviral infection pathway of the present embodiment is mainly a pathway for TMPRSS2-dependent membrane fusion and it is not an endocytosis pathway, the fifth embodiment is characterized in that cells, to which TMPRSS2-dependent membrane fusion is induced upon the invasion of the coronavirus into the cells, are used as host cells. Herein, as an indicator of the evaluation of efficacy, for example, a 50% infection inhibitory concentration (EC₅₀) or the like may be used, but the indicator is not limited thereto.

As a coronavirus used in the present embodiment, a coronavirus developing respiratory syndrome as a result of the infection therewith can be used. Examples of such a coronavirus may include SARS-CoV, SARS-CoV-2, and MERS-CoV. In addition, the host cells are desirably cells, which express factors necessary for coronaviral infection, such as, for example, receptors on the host cell side that are necessary for the invasion of the coronavirus into the host cells (for example, ACE2 (in the case of SARS-CoV and SARS-CoV-2) and CD26 (in the case of MERS-CoV)) and other factors necessary for induction of membrane fusion (for example, TMPRSS2, etc.). These factors may be expressed endogenously, or may also be expressed by foreign genes. Examples of the host cells that can be used in the fifth embodiment may include: airway epithelial cell-derived cells such as Calu-3 cells and H3255 cells; primary human airway epithelial cells (Non Patent Literature 9); pluripotent stem cell-derived mini bronchi (https://www.oita- press.co.jp/1002000000/2020/06/03/NP2020060301000767); and bronchial organoids (https://www.bdr.riken.jp/jp/research/labs/morimoto-m/index.html).

In the screening method according to the fifth embodiment, in addition to a screening system using a DSP assay system, live viruses, or virus like particles (VLP) that retain an ability to invade host cells and do not have pathogenicity, and pseudoviruses may also be used. In the case of using live virus, the presence or absence of virus invasion can be evaluated using the cytopathic effect (CPE) of the host cells, etc. as an indicator. In the case of using VLP, the VLP is allowed to carry a fluorescent dye or the like, and based on whether or not the fluorescence is localized on the host cell membrane or in the cell, the presence or absence of virus invasion can be evaluated. In addition, in the case of using pseudovirus, the pseudovirus is allowed to carry a luciferase gene or the like, and the luciferase activity in the cell is then measured, so that the presence or absence of virus invasion can be evaluated.

When the present description is translated in English and the translation document includes singular terms with the articles "a," "an," and "the,", these terms include not only single items but also multiple items, unless otherwise clearly specified from the context.

Hereinafter, the present invention will be further described in the following examples. However, these examples are only illustrative examples of the embodiments of the present invention, and thus, are not intended to limit the scope of the present invention.

### Examples

### I. Confirmation of membrane fusion inhibitory effect of nafamostat mesylate

### 1. Materials and Methods

### 1-1. Protease inhibitors and anticoagulants

Nafamostat mesylate (Tokyo Chemical Industry Co., Ltd., Tokyo, Japan); camostat mesylate (FUJIFILM Wako Pure Chemical Corporation, Tokyo, Japan); gabexsate mesylate (Tokyo Chemical Industry Co., Ltd.), edoxaban, apixaban, rivaroxaban, and dabigatran (all of which are manufactured by Selleck Chamical Industry, Houston, TX, USA); argatroban (Tokyo Chemical Industry Co., Ltd., Tokyo, Japan); and darexaban (Santa Cruz Biotechnolgy, SantaCruz, CA, USA).

### 1-2. Cell lines and transient gene transfection

In the present example, cell lines, which are based on HEK293FT (a human embryonic kidney-derived immortalized cell line) and each express two split reporters (DSP1-7 and DSP8-11), were used (Wang et al., PLoS ONE 9:e96790 2014). These cell lines (i.e. a cell line expressing DSP1-7 and a cell line expressing DSP8-11) were maintained in Dulbecco's modified Eagle medium (DMEM) supplemented with 10% fetal bovine serum (FBS) and 1 µg/mL puromycin. In order to establish cell lines stably expressing the S protein of SARS-CoV, the S protein of SARS-CoV-2, and the S protein of MERS-CoV, HEK293T cells retaining an S protein-expressing lentivirus transfer plasmid, a psPAX2 packaging plasmid and a vesicular stomatitis virus (VSV)-G-expression plasmid were used to produce a recombinant pseudo-type lentivirus. In order to establish cell lines stably expressing ACE2 or CD26 and TMPRSS2, plat-E cells retaining a VSV-G-expression plasmid were used to produce a recombinant pseudo-type retrovirus (Kitamura et al., Exp. Hematol. 31:1007-1014 2003). 293FT-derived reporter cells that had been infected with the pseudo-type retrovirus were cultured in the presence of 1 µg/mL puromycin, 10 µg/mL blasticidin and 300 µg/mL hydromycin for at least 1 week, and were then selected. The thus selected cells were used in a fusion assay.

Calu-3 cells (ATCC HTB-55) and H3255 cells (CVCL_6831) were airway epithelial cells derived from immortalized cells, which had been established from human lung cancer. These two cell lines were used as target cells (i.e. cells to be infected with virus) in a fusion assay and a viral infection assay.

In order to produce ACE2 knock-out Calu-3 cells, a lentivirus was prepared by transfection with a lentiCRISPRv2 vector (#52961 Addgene, Watertown, MA, USA) retaining the following gRNA sequences. The sequences of the gRNA used herein are as follows: 5'-GCT TTC ACG GAG GTT CGA CG (SEQ ID NO: 1) and 5'- ATG TTG CAG TTC GGC TCG AT (SEQ ID NO: 2) used as controls, and 5'-ATG AGC ACC ATC TAC AGT AC (SEQ ID NO: 3) and 5'-TGC TGC TCA GTC CAC CAT TG (SEQ ID NO: 4) used for knocking out of ACE2. Calu-3 cells infected with pseudo-type virus were selected in the presence of 1 µg/ml puromycin.

### 1-3. Construction of expression vectors

Expression vectors for CD26 and TMPRSS2 were produced according to the previous report (Non Patent Literature 8). An expression vector for ACE2 was produced by cloning the ACE2 gene into a pMXs-internal ribosome entry site (IRES)-blasticidin retroviral vector (Shen et al., Biochimie 142:1-10 2017.). An expression vector for SARS-CoV-2 S protein was constructed by cloning the coding region of the S gene of SARS-CoV-2 (NC_045512.2) (Fasmac, Kanagawa, Japan) into a lentivirus transfer plasmid (CD500B-1, SBI, Palo Alto, CA, USA). An expression vector for SARS-CoV S protein was constructed by cloning the coding region of the S gene of SARS-CoV (NC_004718.3) (Fasmac, Kanagawa, Japan) into a lentivirus transfer plasmid (CD500B-1, SBI, Palo Alto, CA, USA). An expression vector for MERS-CoV S protein was constructed by cloning the coding region of the S gene of MARS-CoV (EMC 2012) into a lentivirus transfer plasmid (CD500B-1, SBI, Palo Alto, CA, USA).

### 1-4. DSP assay for monitoring membrane fusion

In a DSP assay using 293FT cells, effector cells expressing DSP8-11 and S protein, and target cells expressing DSP1-7, CD26 or ACE2, and TMPRSS2, were seeded on a 12-well cell culture plate one day before the assay (2 × 10⁵ cells/500 µL). Two hours before the DSP assay, in order to activate EnduRen as a substrate of renilla luciferase, the cells were treated with 6 µM EnduRen (Promega, Madison, WI, USA). Various types of protease inhibitors or anticoagulants (1 µL each) dissolved in DMSO (dimethyl sulfoxide) were each added into a 384-well plate (Greiner Bioscience, Frickenhausen, Germany). Subsequently, 50 µL each of single cell suspensions (the effector cells and the target cells) removed from the plate by pipetting were added to the wells, using Multidrop Dispenser (Thermo Fisher Scientific, Waltham, MA, USA). After incubation at 37°C for 4 hours, RL activity was measured using Centro xS960 Luminometer (Berthold, Germany).

In a DSP assay using Calu-3 cells or H3255 cells, the target cells were seeded on a 384-well plate one day before the assay (2 × 10⁴ cells/50 µL). Two hours before the DSP assay, the cells were treated with 6 µM EnduRen. Various types of protease inhibitors or anticoagulants (1 µL each) dissolved in DMSO were each added together with 9 µL of a medium into a 384-well plate (Greiner Bioscience, Frickenhausen, Germany). Subsequently, 40 µL of single cell suspension of the aforementioned effector cells, which was treated in the same manner as that described in the above [0049], was added to the wells, using Multidrop Dispenser.

### 1-5. Western blotting

A Western blot analysis was carried out according to the previous report (Yamamoto et al., Commun. Biol. 2019, 2, 292, doi: 10.1038/s42003-019-0547-7.). As primary antibodies, a rabbit anti-ACE2 antibody (1 : 1000, ab15348 Abcam, Cambridge, MA, USA) and an anti-GAPDH antibody (1 : 1000, sc-25778 Santa Cruz Biotechnology, Dallas, TX, USA) were used. An HRP-bound donkey anti-rabbit IgG antibody (NA934, GE Healthcare, Piscataway, NJ, USA) was used as a secondary antibody.

### 1-6. Isolation of SARS-CoV-2

Vero E6 cells (ATCC CRL-1586) were maintained in an Eagle's minimal essential medium (MEM) supplemented with 10% FBS. The cells were incubated in a 5% CO₂ concentration at 37°C, and then, the presence or absence of contamination by Mycoplasma was constantly examined by a PCR method, so that the cells were confirmed to be free of Mycoplasma. Respiratory organ swabs were obtained from patients who had been hospitalized in Center Hospital of the National Center for Global Health and Medicine and had been confirmed to be infected with COVID-19 by tests. The swabs were provided to Division of Virology, Department of Microbiology and Immunology, the Institute of Medical Science, the University of Tokyo, for infection of Vero E6 cells with the virus and the subsequent isolation of the virus. The experimental protocols were approved by Medical Research Ethics Committee, the University of Tokyo. The SARS-CoV-2 virus was allowed to proliferate in Vero E6 cells that were cultured at 37°C in Opti-MEM I (Invitrogen) containing 0.3% bovine serum albumin (BSA) and 1 µg/mL L-1-tosylamide-2-phenylethylketone (TPCK)-treated trypsin. All of the experiments regarding SARS-CoV-2 were carried out in a reinforced containment laboratory with biosafety level 3 (BSL3) in the University of Tokyo. It is to be noted that the use of this laboratory was approved by Ministry of Agriculture, Forestry and Fisheries, Japan.

### 1-7. In vitro SARS-CoV-2 infection experiment according to TCID50 method

Calu-3 (ATCC HTB-55) cells were maintained in MEM supplemented with 10% FBS. VeroE6/TMPRSS2 cells (Non Patent Literature 10) (JCRB 1819) were allowed to proliferate in DMEM supplemented with 10% FBS in the presence of 1 mg/mL Geneticin (G418; Invivogen) and 5 µg/mL plasmocin prophylactic (Invivogen). All of the cells were incubated in a 5% CO₂ concentration at 37°C, and then, the presence or absence of contamination by Mycoplasma was constantly examined by a PCR method, so that the cells were confirmed to be free of Mycoplasma. The target cells, namely, VeroE6/TMPRSS2 cells or Calu-3 cells were not treated (i.e. without pretreatment) or were pretreated with nafamostat mesylate (10-fold dilution series from 100 µM to 10 nM, 4 wells for each concentration) for 1 hour (i.e. with pretreatment). Thereafter, SARS-CoV-2 was added to the resulting cells to a multiplicity of infection (MOI) of 0.01 or 0.1. In order to infect the cells with the virus, the virus and the cells were incubated for 30 minutes, and the medium was then exchanged with a new culture medium containing the same concentration of nafamostat mesylate. Based on the form of cells by a visually detectable cytopathic effect (CPE), the IC₅₀ value was determined according to the Spearman-Karber calculation formula, 3 days after the infection in the case of the VeroE6/TMPRSS2 cells, and 5 days after the infection in the case of the Calu-3 cells.

### 1-8. In vitro SARS-CoV-2 infection experiment according to q-PCR method

Calu-3 (ATCC HTB-55) cells were maintained in MEM supplemented with 10% FBS. In an experiment with pretreatment, the Calu-3 cells were pretreated with nafamostat mesylate (10-fold dilution series from 100 µM to 0.01 nM, 3 wells for each concentration) for 1 hour, and then, SARS-CoV-2 was added to the resulting cells to a multiplicity of infection (MOI) of 0.01. In order to infect the cells with the virus, the virus and the cells were incubated for 30 minutes, and the medium was then exchanged with a new culture medium containing nafamostat mesylate. On the other hand, in an experiment without pretreatment, SARS-CoV-2 was added to the Calu-3 cells to a multiplicity of infection (MOI) of 0.01, and the virus and the cells were then incubated for 30 minutes. Thereafter, the medium was exchanged with a new culture medium containing nafamostat mesylate (10-fold dilution series from 10 µM to 1 nM, 3 wells for each concentration). Twenty-four hours after the infection, the medium was removed, and the cells were washed once with 100 µl of PBS. After that, 120 µl of Lysis Solution (SuperPrepII Cell Lysis & Kit for RT Kit for qPCR, Toyobo) was added to the resultant, and the obtained mixture was then incubated at room temperature for 5 minutes, so as to prepare a cell lysate. Using 4 µl of the cell lysate, 20 µl of reverse transcription reaction solution (SuperPrepII Cell Lysis & Kit for RT Kit for qPCR, Toyobo) was prepared, and cDNA was then synthesized according to a reverse transcription reaction. The amounts of SARS-CoV-2 N gene cDNA and glyceraldehyde-3-phosphate dehydrogenase (GAPDH) gene cDNA in the reaction solution were measured according to real-time PCR using CFX Connect Real-Time PCR Detection System (Bio-Rad). As primers used in the PCR reaction, 5'-AAA TTT TGG GGA CCA GGA AC-3' (SEQ ID NO: 5) and 5'- TGG CAG CTG TGT AGG TCAAC-3' (SEQ ID NO: 6) were used for detection of the SARS-CoV-2 N gene cDNA, and 5'-GCA CCG TCA AGG CTG AGA AC-3' (SEQ ID NO: 7) and 5'-TGG TGA AGA CGC CAG TGG A-3' (SEQ ID NO: 8) were used for detection of the GAPDH gene cDNA. The amount of the SARS-CoV-2 N gene cDNA was corrected with the amount of the GAPDH gene cDNA. Using such data, the values of IC₅₀, IC₉₀, and IC₉₉ were calculated according to a Probit method.

### 2. Results

### 2-1. Studies regarding influence of TMPRSS2 inhibitor on membrane fusion using DSP reporter assay

In DSP reporter assay performed in the present example, a chimeric protein (DSP1-7), in which the N-terminal fragment of Renilla luciferase (RL) binds to the N-terminal fragment of GFP, and a chimeric protein (DSP8-11), in which the C-terminal fragment of GFP binds to the C-terminal fragment of RL, were each allowed to express in different cells. A phenomenon, in which the two proteins meet for the first time when those cells take place membrane fusion and the proteins form a complex, is utilized (Figure 1A). The two proteins emit neither fluorescence nor luminescence alone, but if the two proteins form a complex as a result of the membrane fusion, the complex emits fluorescence and luminescence (Figures 1A and B). In the following example, fusion was quantified by luminescence, because of its favorable quantitativity and sensitivity.

First, using 293FT cells that did not endogenously express ACE2 and TMPRSS2, the ACE2/TMPRSS2 dependence of membrane fusion was studied. When target cells (corresponding to host cells in actual viral infection) prepared by allowing DSP1-7-expressing 293FT cells (i.e. 293FT-DSP1-7) to further express both ACE2 and TMPRSS2, were mixed with effector cells (corresponding to virus in actual viral infection) prepared by allowing 293FT-DSP8-11 to express SARS-CoV-2 S protein (Figure 2A) or SARS-CoV S protein (Figure 5), membrane fusion was induced in both cells (Figure 2A and Figure 5; the 5th column). In cells in which ACE2 alone (Figure 2A and Figure 5; the 3rd column) or TMPRSS2 alone (Figure 2A and Figure 5; the 4th column) was expressed, membrane fusion hardly occurred. In addition, even though both ACE2 and TMPRSS2 were expressed, membrane fusion did not occur, unless the S protein was present (Figure 2A and Figure 5; the 2nd column). These results demonstrate that membrane fusion induced by the S proteins of SARS-CoV and SARS-Cov-2 is apparently dependent on three substances, namely, ACE2, TMPRSS2 and the S protein.

Moreover, when cells, which were prepared by knocking out ACE2 in Calu-3 cells endogenously expressing ACE2 and TMPRSS2 by a CRISPR-Cas9 system, were used as target cells, membrane fusion hardly occurred (Figure 2B; the 5th and 6th columns). Accordingly, it was suggested that functional receptors of SARS-CoV-2 should not be present, other than ACE2, in certain types of lung epithelial cells.

Using a system in which 293FT cells were used as target cells, whether or not the existing three pharmaceutical agents used in the treatment of pancreatitis and/or DIC (nafamostat mesylate, camostat mesylate, and gabexate mesylate) inhibit membrane fusion induced by the S protein of SARS-CoV-2 was studied. These existing pharmaceutical agents are serine protease inhibitors.

Nafamostat mesylate (concentration: 0.01 µM to 1 µM) exhibited the highest inhibitory effect on membrane fusion (Figure 3; the 6th to 8th columns). The influence of nafamostat mesylate on cell fusion was observed under a phase-contrast microscope. As a result, membrane fusion occurred in the absence of nafamostat mesylate, and multinucleated cells could be confirmed (Figure 4, center). However, it could be confirmed that membrane fusion did not occur in the presence of 1 µM nafamostat mesylate (Figure 4, right), as in the case of not expressing ACE2 and TMPRSS2 (Figure 4, left). With regard to serine protease inhibitors other than nafamostat mesylate, it could be confirmed that membrane fusion was inhibited by camostat mesylate (Figure 3; the 9th to 11th columns), but its inhibitory effect was about 1/10 of the inhibitory effect of nafamostat mesylate. The inhibitory effect of gabexsate mesylate on membrane fusion could not be confirmed, even if gabexsate mesylate was used, at least, in the concentration range examined herein (1 µM to 10 µM) (Figure 3; the 4th and 5th columns).

Subsequently, using a system in which 293FT cells were used as target cells (Figure 5), the inhibitory effects of nafamostat mesylate, camostat mesylate and gabexsate mesylate on the membrane fusion induced by the S protein of SARS-CoV were also examined.

Nafamostat mesylate exhibited the highest inhibitory effect on membrane fusion, in the concentration range of 0.1 µM to 10 µM (Figure 6; the 6th to 8th columns), and camostat mesylate exhibited the second highest inhibitory effect (Figure 6; the 9th to 11th columns). As in the case of membrane fusion by SARS-CoV-2, the inhibitory effect of gabexsate mesylate on membrane fusion could not be confirmed, even if gabexsate mesylate was used in the concentration range examined herein (1 µM to 10 µM) (Figure 6; the 4th and 5th columns).

Furthermore, using airway epithelium-derived Calu-3 cells and H3255 cells as target cells, the influence of nafamostat mesylate and camostat mesylate on the membrane fusion induced by the S protein of SARS-CoV-2 was studied. It is considered that the Calu-3 cells and the H3255 cells endogenously express ACE2 and TMPRSS2, and thus that these cells reflect a physiological environment more strongly than 293FT kidney cells do. As a result of the experiment, it was found that nafamostat mesylate inhibited the membrane fusion induced by the S protein of SARS-CoV-2 in the concentration range of 0.001 µM to 0.01 µM (1 nM to 10 nM) (Figures 7A and B; the 4th and 5th columns). In contrast, in order for camostat mesylate to exhibit the same level of inhibitory effect as that of nafamostat mesylate, the concentration range of camostat mesylate needed to be 0.01 µM to 0.1 µM (10 nM to 100 nM) (Figures 7A and B; the 6th to 8th columns). Accordingly, it is considered that nafamostat mesylate has inhibitory activity against membrane fusion that is about 10-fold stronger than that of camostat mesylate. In addition, the concentration of a serine protease inhibitor necessary for inhibition of the membrane fusion by the S protein of SARS-CoV-2 in airway epithelial cells was about 10-fold lower than the concentration of the serine protease inhibitor necessary for inhibition of membrane fusion in kidney-derived 293FT cells used as target cells (for example, please compare the 6th and 7th columns of Figure 3, with the 4th and 5th columns of Figures 7A and B).

Recently, various computational scientific methods have been used in searching for existing pharmaceutical agents that target TMPRSS2 (Rensi et al., ChemRxiv 2020, doi:10.26434/chemrxiv.12009582.v1 (accessed on June 7, 2020); Kim et al., ChemRxiv 2020, doi:10.26434/chemrxiv.12037416.v1 (accessed on June 7, 2020)).

According to the results of the searching by such a computational scientific method, nafamostat mesylate is ranked much higher than camostat mesylate, which corresponds to the results of the present example. Moreover, notably, an anticoagulant that is a serine protease inhibitor targeting Factor Xa or thrombin is highly ranked. Hence, the present inventors have studied whether or not these anticoagulants influence on the membrane fusion induced by the S protein of SARS-CoV-2. However, all of the examined anticoagulants did not exhibit inhibitory effects on the membrane fusion (Figure 8).

### 2-2. Studies regarding inhibitory effect of nafamostat mesylate on infection of VeroE6/TMPRSS2 cells and Calu-3 cells with SARS-CoV-2 according to TCID50 method

According to a DSP assay, nafamostat mesylate was identified as an inhibitory agent of the membrane fusion induced by the SARS-CoV-2 S protein (Figure 3, Figure 4, and Figure 7). This inhibition of the membrane fusion was considered to be the effect obtained by inhibiting TMPRESS2 activity on the cell membrane. Upon infection of Calu-3 cells with SARS-CoV-2, this virus invades the cells mainly via a TMPRSS2-dependent cell membrane pathway (Non Patent Literatures 8 and 9, and Kawase et al., J. Virol. 2012, 86, 6537-6545, doi: 10.1128/JVI.00094-12.). On the other hand, upon infection of VeroE6/TMPRSS2 cells with SARS-CoV-2, this virus invades the cells via a TMPRSS2-independent endocytosis pathway, as well as via the cell membrane pathway (Matsuyama et al., BioRxiv, 2020, doi: 10.1101/2020.03.11.987016. (accessed on 7 June 2020)).

Hence, the effects of nafamostat mesylate on infection of Calu-3 cells and VeroE6/TMPRSS2 cells with SARS-CoV-2 were studied. The effects of nafamostat mesylate were evaluated, both in the case of performing a pretreatment with nafamostat mesylate, and in the case of not performing a pretreatment with nafamostat mesylate.

The degree of infection was evaluated based on the appearance of a cytopathic effect (CPE), and IC₅₀ was then calculated according to the TCID50 method using the Spearman-Karber calculation formula. The IC₅₀ value to the Calu-3 cells was 6.8 to 11.5 nM (with pretreatment) and 3.16 µM (without pretreatment), whereas the IC₅₀ value to the VeroE6/TMPRSS2 cells was 31.6 µM (with pretreatment) and 100 µM or more (without pretreatment) (Figure 9). Since the IC₅₀ value was significantly decreased when the cells were pretreated with nafamostat mesylate, it is suggested that nafamostat mesylate should inhibit invasion of SARS-CoV-2 into the cells.

### 2-3. Studies regarding inhibitory effect of nafamostat mesylate on infection of Calu-3 cells with SARS-CoV-2 according to q-PCR method

In order to more quantitatively evaluate the inhibitory effect of nafamostat mesylate on infection of Calu-3 cells with SARS-CoV-2, studies were conducted according to a q-PCR method. As a result, in the experiment with pretreatment, IC₅₀ = 0.27 nM, IC₉₀ = 1.6 nM, and IC₉₉ = 6.5 nM (Figure 10A). On the other hand, in the experiment without pretreatment, IC₅₀ = 0.97 nM, IC₉₀ = 15 nM, and IC₉₉ = 1500 nM (1.5 µM) (Figure 10B).

More importantly, when Calu-3 cells pretreated with nafamostat mesylate were used, the cells exhibited extremely high sensitivity to nafamostat mesylate, having IC50 = 0.27 nM, IC₉₀ = 1.6 nM, and IC₉₉ = 6.5 nM. From these results, a TMPRSS2-dependent pathway was considered to be a main infection pathway of SARS-CoV-2 in airway epithelium-derived Calu-3 cells, and thus, unexpected results were obtained, which were not found in the study paper of Wang et al., in which the effects of nafamostat mesylate on infection with SARS-CoV-2 were examined using Vero E6 cells, in which TMPRSS2 was not expressed, and the results determining that nafamostat mesylate has "no effects" were obtained.

### II. Combined administration of nafamostat mesylate and favipiravir to severe COVID-19 patients

### 1. Methods

### 1-1. Subjects

Twelve adults who had been confirmed to be infected with SARS-CoV-2 according to an RT-PCR method had been hospitalized in the intensive care unit (ICU) of the University of Tokyo Hospital from April 6, 2020 to April 21, 2020. The test samples were collected from nasopharynx swabs or by intratracheal aspiration. A combination therapy using both favipiravir and nafamostat mesylate was performed on 11 out of the 12 patients who had been confirmed to be infected with SARS-CoV-2. The demographic data, and clinical symptoms and main symptoms, and clinical test results obtained during hospitalization in ICU were collected from their electronic health records. All of the clinical tests and radiological tests were carried out at the discretion of doctors who treated the patients.

### 1-2. Statistical analysis

The continuous variable is indicated with the median and the interquartile range (IQR). In addition, the categorical variable is indicated with number and ratio.

### 2. Results

The demographic and clinical characteristics of the patients are shown in Table 1.

**[Table 1]**

| | **Characteristics** | **Values** |
|---|---|---|
| Age; median (IQR), years old | | 68 (60-69) |
| Male; number of people (%) | | 10 (91%) |
| Body weight; median (IQR), kg | | 71 (69-82) |
| Number of patients with complication; number of people (%) | | |
| | Asthma | 0 (0%) |
| | Cancer | 1 (9%) |
| | Chronic kidney disease | 0 (0%) |
| | Chronic obstructive pulmonary disease | 1 (9%) |
| | Diabetes | 3 (27%) |
| | Hypertension | 4 (36%) |
| Symptom duration before hospitalization; median (IQR), number of days | | 8 (7-11) |
| Number of patients with various symptoms; number of people (%) | | |
| | Fever | 9 (82%) |
| | Cough | 5 (45%) |
| | Breath shortness | 8 (73%) |

The median of the patient's ages was 68 years old (IQR, 60-69 years old), and male patients accounted for 91%. Four patients (36%) had hypertension, and 3 patients (27%) had diabetes. In addition, 1 patient (9%) had chronic obstructive pulmonary disease.

Clinical examination values and CT examination findings during hospitalization in ICU are shown in Table 2.

**[Table 2]**

| **Clinical Examination Values** | |
|---|---|
| Leukocyte count; median (IQR), per mm³ | 6900 (5800-10850) |
| Lymphocyte count; median (IQR), per mm³ | 851 (759-1164) |
| Hemoglobin; median (IQR), g/dl | 15.0 (13.5-16.3) |
| Platelets; median (IQR), per mm³ | 19.6 (18.6-26.4) |
| Lactate dehydrogenase; median (IQR), U/liter | 518 (417-752) |
| Aspartate aminotransferase; median (IQR), U/liter | 54 (49-90) |
| Alanine aminotransferase; median (IQR), U/liter | 47 (35-58) |
| Serum creatinine; median (IQR), mg/dl | 0.85 (0.70-1.03) |
| Creatinine kinase; median (IQR), U/liter | 213 (129-579) |
| Prothrombin time; median (IQR), international normalized ratio | 1.08 (1.03-1.13) |
| Activated partial thromboplastin time; median (IQR), sec. | 28.8 (28.4-32.1) |
| D-dimer value; median (IQR), ug/dl | 1.4 (1.1-11.8) |
| PaO₂/FiO₂ ratio; median (IQR) | 131 (114-198) |
| SOFA score; median (IQR) | 3.0 (2.5-4.5) |
| APACHE II score; median (IQR) | 14.0 (12.0-15.5) |

| **CT Examination Findings** | |
|---|---|
| Patients with induration; number of people (%) | 6 (55%) |
| Patients with ground-glass opacity; number of people (%) | 10 (91%) |
| Patients with infiltration of the lungs; number of people (%) | 2 (18%) |

| | |
|---|---|
| APACHE: Acute Physiology and Chronic Health Evaluation FiO₂: fraction of inspired oxygen (fraction of inspiratory oxygen) PaO₂ partial pressure of arterial oxygen (partial pressure of oxygen in arterial blood) SOFA : sequential organ failure assessment | |

An increase in lactate dehydrogenase values was found in all of the patients. Lymphocytic leukopenia (< 1000 per mm³) was found in 6 patients (55%), and an increase in D-dimer values (> 2.0 µg per dl) was found in 5 patients (45%). Computed tomography (CT scan) was performed on individual patients. Ten patients (91%) showed ground-glass opacities, and 6 patients (55%) showed lung induration.

Nafamostat mesylate was administered to the patients at a dose of 0.2 mg/kg/hour via continuous intravenous drip. Favipiravir was administered to the patients at doses of 3600 mg (Day 1) and then 1600 mg (on and after Day 2) orally or using a gastric tube. The median of the treatment periods with nafamostat mesylate was 14 days (IQR, 10-14 days), and the median of the treatment periods with favipiravir was 14 days (IQR, 12-14 days). Except for one patient in whom hyperkalemia was observed on Day 9, the antiviral therapy was not suspended due to side effects.

Observation of the progress of all of the 11 patients was carried out by the hospital for at least 33 days. On May 7, 2020, one patient with "do not resuscitate order" died on Day 7 after hospitalization in ICU. However, 9 patients were discharged from the ICU, and 7 patients left the hospital (Figure 11). According to the report of the Japanese Society of Intensive Care Medicine, the therapeutic results of COVID-19 in Japan were good compared with those in foreign countries, and in the case of patients wearing artificial ventilator (non ECMO), the mortality was 19%, and the percentage of the survivors from ECMO was 73.6% (https://www.covid19-jma-medical-expert- meeting.jp/topic/1121). Even compared with these results, the administration results of nafamostat mesylate and favipiravir are considered to be excellent.

The results of the present clinical trial are initial reports regarding a combination therapy of nafamostat mesylate and favipiravir. The percentage of seriously ill patients who need the wearing of IMV was high in the present clinical trial (8 patients; 73%). Nevertheless, mortality was extremely low (one patient with "do not resuscitate order"; 9%). Seriously ill COVID-19 patients often exhibit alveolar shadow in the lung, microvascular thrombosis attended with interstitial inflammation, and bleeding symptoms (McGonagle et al., Lancet Rheumatol. 2020, doi:10.1016/S2665-9913(20)30121-1). Since nafamostat mesylate inhibits intravascular coagulation and directly inhibits invasion of the virus into host cells, nafamostat mesylate is considered to be extremely effective for the treatment of COVID-19.

It is conceived that combined administration of nafamostat mesylate and favipiravir inhibits both invasion of the virus into cells and replication of the virus in the cells, and at the same time, the combined administration is able to suppress various symptoms (for example, severe coagulation abnormalities) caused by the viral infection.

### Industrial Applicability

The present invention provides a therapeutic agent or a therapeutic composition for viral infection, for example, viral infection caused by SARS-CoV, SARS-CoV-2 and the like. Therefore, it is greatly expected that the present invention will be utilized in the medical field.

## Claims

1. An inhibitory agent of membrane fusion between a virus envelope and a cell membrane of a host cell for the virus, wherein the inhibitory agent comprises a serine protease inhibitor.

2. The inhibitory agent according to claim 1, wherein TMPRSS2 activity is required for invasion of the virus into the host cell.

3. The inhibitory agent according to claim 1 or 2, wherein the virus is SARS-CoV-2 or SARS-CoV.

4. The inhibitory agent according to any one of claims 1 to 3, wherein the serine protease inhibitor is one or more of a compound represented by the following general formula (I) or a salt thereof, or a solvate or hydrate thereof: 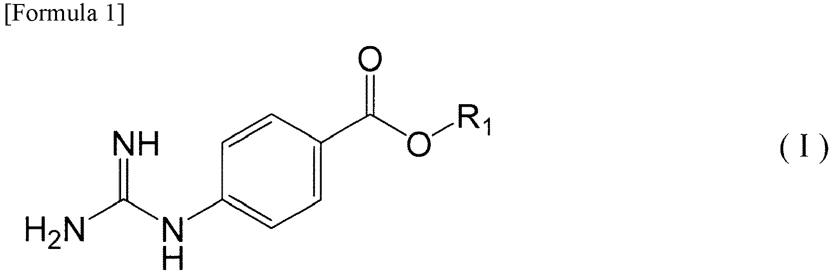 wherein R₁ represents a substituted or unsubstituted aromatic hydrocarbon group, a substituted or unsubstituted aromatic heterocyclic group, a substituted or unsubstituted lower alkyl group, or a substituted or unsubstituted lower alkenyl group, each of which may optionally comprise a heteroatom.

5. The inhibitory agent according to claim 4, wherein R₁ represents an aromatic hydrocarbon group represented by the following formula (1) or (2): 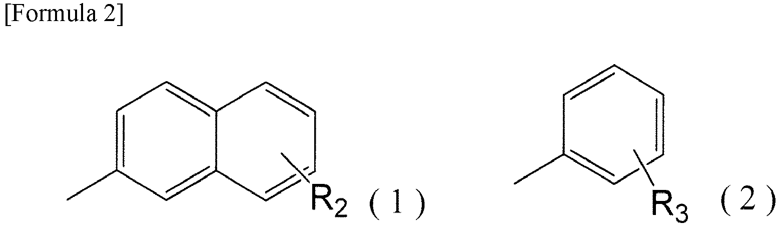 wherein, in the above formulae (1) and (2), R₂ and R₃ each independently represents a lower alkyl group optionally having a heteroatom and/or a substituent.

6. The inhibitory agent according to claim 5, wherein R₂ represents a substituent represented by the following formula (3), and R₃ represents a substituent represented by the following formula (4):

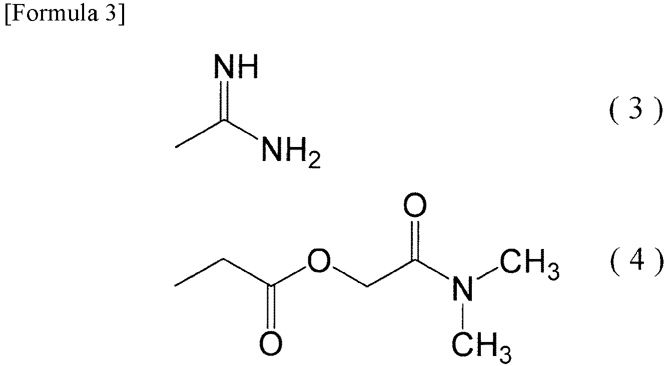

7. The inhibitory agent according to claim 4, wherein the compound represented by the general formula (I) is nafamostat and/or camostat.

8. A therapeutic agent or a therapeutic composition for viral infection, wherein the therapeutic agent or the therapeutic composition comprises, as an active ingredient, the inhibitory agent according to any one of claims 1 to 7.

9. The therapeutic agent or the therapeutic composition according to claim 8, wherein the virus is SARS-CoV-2 or SARS-CoV.

10. The therapeutic composition according to claim 9, which is an injection, a tablet, or an inhalant.

11. A method for evaluating the efficacy of an inhibitory agent of coronaviral infection, wherein the method is **characterized in that** a cell that is mainly TMPRSS2-dependently infected with the virus via a membrane fusion pathway is used as a host cell.

12. The method according to claim 11, which is **characterized in that** the coronavirus is SARS-CoV, SARS-CoV-2, or MERS-CoV.
